# EUROPEAN PATENT APPLICATION

(11) **EP 2 283 805 A1**
(43) Date of publication of application: **16.02.2011**
(21) Application number: 09166660.2
(22) Date of filing: 28.07.2009
(51) Int. Cl.: A61K 8/27, A61K 9/06, A61K 33/30, A61Q 1/04, A61Q 19/00

(54) **Compositions comprising a zinc containing compound dissolved in a hydrophobic phase**

(71) Applicant: Sirvis BV, 1015 NV Amsterdam (NL)
(72) Inventor: Brand-Garnys, Elzbieta Ewa, 2914 AD, Nieuwenkerk aan den IJssel (NL)
(74) Representative: Farago, Peter Andreas

(57) **Abstract**

The invention relates to compositions, methods of preparing compositions, treatment usages, dispensers and method of manufacturing dispensers. Examples of such compositions are lipsticks, lip balm, lip ointments and lip gloss. The compositions preferably comprise a zinc containing compound dissolved in a hydrophobic phase. The zinc containing compound has an anti-herpetic effect and can be used for treatment or prevention of virus infections, particularly relating to the lips.

## Description

### Field of the invention

The present invention relates to the field of lip compositions. Examples of such compositions include lipsticks, lip balm and lip gloss.

### Background & Prior Art

Medical Hypotheses, vol. 17, pp. 157-165 (1985) discusses the beneficial use of zinc ions in aqueous environments. GB 2306324 discloses aqueous anti-herpes compositions comprising acyclovir and several zinc ions. EP000133 and EP012115 disclose aqueous compositions for treating herpes infections comprising sulfated polymers and zinc ions. US4661354 discloses aqueous compositions comprising camphor and zinc sulfate for topical treatment of herpes simplex infections. WO94021 discloses powered zinc glycerolate compositions for treatment of herpetic infections. US4762715 relates to anti-herpetic lipstick formulations comprising dissociable zinc sulfate heptahydrate. WO996381 discloses compositions with zinc compounds in hydrophilic glycerin for use against herpetic infections. WO 2004/066971 describes aqueous compositions comprising zinc ions as anti-viral agent. WO0062738 discloses lipsticks with antibacterial zinc compound. In the examples, zinc citrate is combined in one phase with menthol and anethole, indicating a low processing temperature. The lipsticks contain water to deliver the zinc ions to the oral cavity, resulting in a wash-off effect from the lips. DE4425322 describes dermatological products comprising acyclovir and/or zinc compounds as active ingredients. The active ingredients are present in particulate form, leading to a gritty product, requiring extensive milling, and leading to suboptimal clinical results.

Compositions disclosed in the art suffer from drawbacks in that they are not always sufficiently effective in prevention and/or treatment of viral infections, are chemically and/or physically unstable, and/or cause side-effects.

We have further found that compositions containing water-soluble zinc sulfate have a watery feel which is not appreciated by users for various reasons, including greater difficulty in precisely applying lip products, larger wash-off effect of lip products, leading to lower efficacy.

Use of many lip compositions of the art result in esthetically displeasing look and feel of the lips. For instance, certain compositions give a -not preferred- gritty appearance and feel to the lips which is preferably avoided in view of the importance of kiss appeal of lip products. To avoid this gritty appearance, prior art disclosures would require solid particle grinding, requiring time and energy, while often not even resulting in an optimal end product. Astringent side-effects of zinc have been described. We have found that particles containing zinc may in particular cause these undesirable astringent effects, possibly due to the particles causing higher, localized concentrations of zinc.

We have found that the above disadvantages of the prior art can be overcome. In particular, we have found that the invention is directed to lip compositions that provide excellent anti-herpetic activity, while being chemically and/or physically stable, cause no/fewer side-effects, provide excellent look and lip feel, with low wash-off effects and high kiss appeal.

### Disclosure of the invention

According to one embodiment, the present invention relates to compositions comprising a zinc containing compound dissolved in a hydrophobic phase.

In another embodiment, the present invention relates to a method of preparing a composition comprising a hydrophobic phase and a zinc containing compound, wherein the zinc containing compound is dissolved in the hydrophobic phase at an elevated temperature, wherein the composition is cooled down and wherein the composition comprises less than 15% by weight of water.

In another embodiment, the present invention relates to a hydrophobic phase-dissolved zinc containing compounds for use in preventing and/or treating of lip sores.

In another embodiment, the present invention relates to a dispenser device comprising a container reservoir and a lid, wherein the reservoir comprises a composition comprising a zinc containing compound dissolved in a hydrophobic phase.

In another embodiment, the present invention relates to a method of manufacturing a dispenser device with a composition comprising a zinc containing compound dissolved in a hydrophobic phase , wherein the device is filled with the composition at a temperature of from 30°C and up to 65°C.

In another embodiment, the present invention relates to a composition comprising zinc containing compound and zinc oxide particles.

We have found that the compositions of the invention overcomes the above-mentioned problems of the prior art. In addition, compositions of the invention show high and improved bioavailability which is important in treatment of herpetic infections. One element in achieving such bioavailability is believed to be the hydrophobic phase of the formulation; another element is believed to be the presence of zinc containing compound, particularly in a dissolved state; yet another element is presence of zinc oxide particles; and a further element is the presence of a structurant.

### Hydrophobic phase

Preferably, compositions of the present invention comprise a hydrophobic phase, more preferably a hydrophobic continuous phase. Preferably, the hydrophobic phase comprises one or more compounds selected from a fatty compound, a wax, an emulsifier and mixtures thereof.

Fatty compounds (also defined as oils and butters) are preferably selected from:
(1) fatty acid esters preferably including including saturated and unsaturated fatty acids and preferably including oleochemical and petrochemical esters, more preferably derived from the esterification reaction of C8-C20 fatty acids with C1-C20 monohydric and polyhydric alcohols (for example triglycerides), most preferably derived from caprylic, capric, adipic, diisopropyl adipate, lauric, myristic, palmitic, stearic, hexadecyl stearate, hydrogenated vegetable oils, behenic, isostearic, oleic, isopropyl palmitate, isopropyl myristate, sebacate, diethyl sebacate and erucic acid, esterified with suitable mono- or polyhydric alcohols; illustrative but not limiting examples include cetyl ricinoleate, glycerol oleate, glycerol monostearate, isopropyl lanolate, isopropyl linoleate, isopropyl myristate, ethyl palmitate, isopropyl palmitate, isopropyl isostearate, and mixtures thereof;
(2) liquid wax esters, preferably liquid up to a temperature of 45°C, more preferably liquid at 20°C; preferably selected from jojoba oil, orange roughy oil, lanolin (Adeps Lanae), and mixtures thereof;
(3) triglyceride oils, preferably selected from almond oil, apricot kernel oil, avocado oil, babassu kernel oil, castor oil, coconut oil, cotton seed oil, corn oil, evening primrose oil, grape seed oil, kukui nut oil, olive oil, palm kernel oil, peach kernel oil, pleasure oil, rapeseed oil, safflower seed oil, sesame oil, soybean oil, sunflower oil, wheat germ oil, various other vegetable triglyceride oils, and mixtures thereof;
(4) butters preferably triglycerides, more preferably triglycerides that melt around body temperature, most preferably selected from shea butter, illipe butter, murumuru butter, cocoa butter, shorea butter, and mixtures thereof;
(5) hydrocarbon oils may be either natural or synthetic; preferably liquid up to a temperature of 45°C; preferably derived from mineral sources include petroleum derived mineral oils, petrolatum and mixtures thereof; more preferably polybutene;
(6) volatile and non-volatile silicone oils, such as dimethicone and cyclomethicone;
(7) fatty alcohols preferably selected from cetearyl alcohol, cetyl alcohol (cetyl oleate), myristyl alcohol, stearyl alcohol, isostearyl alcohol, lanolin alcohol, lauryl alcohol, oleyl alcohol, hexadecyl alcohol, and mixtures thereof; and
(8) mixtures thereof.

Wax is preferably selected from hard waxes with a melting point of preferably higher than 45°C, more preferably higher than 55°C, most preferably higher than 60°C and preferably up to 110°C; derivatives of said waxes whereby the free acids are esterified with a suitable mono- or polyhydric alcohol; preferably waxes are selected from the group consisting of animal waxes, vegetable waxes, mineral waxes, synthetic waxes, and mixtures thereof; the waxes preferably have a melting point of higher than 55°C and preferably up to 100°C and a needle penetration, as measured according to the American standard ASTM D5, of 3 to 40 at 25°C; more preferably waxes are selected from animal (preferably beeswax, spermaceti, lanolin, shellac wax), vegetable (preferably Carnauba, candellila, bayberry, sugarcane wax), mineral (preferably ozokerite, ceresin, montan, paraffin, microcrystalline, petroleum and petrolatum wax) and synthetic waxes (preferably silicone waxes, polyol ether-esters, hydrogenated plant oils such as castor oil, jojoba wax, sunflower oil, cotton oil, coprrac oilik and lanolin oils-; ethylenic polymers, and hydrocarbon type wax, such as Fischer Tropsch waxes); most preferably wax is selected from beeswax, lanolin wax, carnauba wax (Copernicia Cerifera), candelilla wax (Euphorbia Cerifera), bayberry wax, ozokorite, ceresin, paraffins, microcrystalline waxes, polyethylene, C24-45 alkyl methicones, orange wax, hard waxes with a melting point between 60-110°C, derivatives of said waxes whereby the free acids are esterified with a suitable mono- or polyhydric alcohol, and mixtures thereof.

Emulsifiers (or wetting agents) are preferably selected from the range of emulsifiers with a HLB value in the range of 1-18, preferably 2-13 and best 3-8.

Preferably, the composition comprises at least 50%, more preferably at least 60%, most preferably at least 75% and preferably at most 98%, more preferably at most 92%, most preferably at most 90% by weight of fatty compound. Preferably, the composition comprises at least 0.1%, more preferably at least 2%, most preferably at least 4% and preferably at most 30%, more preferably at most 25%, most preferably at most 20%, and particularly preferred at most 18%by weight of wax. Preferably, the composition comprises at least 0.2%, more preferably at least 0.5%, most preferably at least 1.5% and preferably at most 10%, more preferably at most 7.5%, most preferably at most 5% by weight of emulsifier.

As indicated above, waxes are solid lipophilic materials preferably have a melting point of from about 55° C, more preferably of from 65°C to about 110 C. These solids include fatty alcohols, fatty acid esters, waxes and mixtures thereof. Substances mentioned above as fatty compounds having a melting point within the above range are considered to wax.

It is preferred that compositions according to the invention comprise at least one wax selected from the waxes identified above. These waxes provides excellent structure and other product characteristics -such as shine and texture- to lip compositions of the present invention, including lip sticks, lip balm and lip gloss formulations. More preferably, compositions of the invention comprise a wax and a fatty compound as indicated above to provide preferred texture at room temperature of 20°C and when used on lips at a temperature of 37°C.

### Zinc containing compound

Compositions according to the invention comprise at least a zinc containing compound. Preferably, the zinc containing compound is selected from zinc containing compounds that are soluble in a hydrophobic phase, preferably at a temperature of below 160 °C.

Preferably, zinc containing compounds according to the invention are anhydrous. While the zinc containing compound may be present as particles in some embodiments of the present invention, preferably, the zinc containing compound is dissolved.

Preferably, the zinc containing compound is selected from zinc ricinoleate (zinc bis-(12-hydroxy-cis-9-octodecenoate), zinc acetylacetonate, zinc gluconate (preferably anhydrous), zinc undecylenate, zinc stearate, zinc palmitate, zinc succinate, zinc lactate (preferably anhydrous), zinc pyrrolidon, zinc bis-(8-hydroxycaprylate), zinc glutamate (preferably anhydrous), zinc malate and zinc tartrate.

More preferably, the zinc containing compound is selected from zinc ricinoleate (12-hydroxy-cis-9-octadecenoic acid), zinc lactate (preferably anhydrous), zinc malate, zinc tartrate, zinc undecylenate and mixture thereof. Most preferably, the zinc containing compound is selected from zinc ricinoleate (12-hydroxy-cis-9-octadecenoic acid), zinc lactate (preferably anhydrous), zinc malate, zinc tartrate and mixture thereof. Particularly preferred (for instance, in view of the electrovalent characteristics and stability) are the zinc containing compounds selected from zinc ricinoleate (12-hydroxy-cis-9-octadecenoic acid), anhydrous zinc lactate, zinc malate, and mixture thereof.

An especially preferred zinc containing compound is zinc di-ricinoleate, often simply referred to as zinc ricinoleate. It can be represented by zinc bis-(12-hydroxy-cis-9-octadecenoic acid) or specifically by [9-octadeceonic acid, 12 hydroxy, zinc salt (2:1), (9Z, 12R) and has formula structure: Zn(O-CO-(CH₂)₇-CH=CH-CH₂-CHOH-(CH₂)₅-CH₃)₂. One commercial source of zinc ricinoleate is TEGO ® Sorb (Goldschmidt AG, Essen, Germany). Zinc ricinoleate is a known deodorizing agent for cosmetic products and it may be produced by alkaline saponification of technical ricinus oil and subsequent reaction with aqueous zinc sulfate solution.

Preferably, the zinc containing compound is dissolved in the hydrophobic phase. The solution of the zinc containing compound in the hydrophobic phase may be liquid or solid, depending on the temperature and preferably solid below 40°C. The invention is preferably directed at a solidified mixture of the zinc containing compound dissolved in the hydrophobic phase. This mixture may be liquid at an elevated temperature, and upon cooling, may turn into a solid solution. Preferably, the zinc containing compound is at least partially in dissolved state, more preferably at least 50% dissolved, most preferably at least 90%, particularly preferred essentially in dissolved state. For the purpose of this invention, the zinc containing compound may be present as a crystalline dispersion in the hydrophobic phase, preferably as a submicron crystalline dispersion (due to crystallization at lower temperatures), more preferably at least as a molecular dispersion in the hydrophobic phase, most preferably primarily -or even particularly preferred substantially entirely- as a molecular dispersion.

Preferably, zinc containing compounds are present in compositions of the invention at levels of from 0.1%, more preferably from 0.5%, most preferably from 1.0% and preferably up to 12%, more preferably up to 8%, most preferably up to 6%, particularly preferred 5% by weight of the composition.

### Zinc oxide

In order to provide longer term anti-herpetic activity, compositions according the invention preferably comprise zinc oxide. Preferably, zinc oxide is present as solid particles suspended in the hydrophobic phase, more preferably zinc oxide is suspended at least partially by means of a polymeric structure. Preferably, zinc oxide provides the longer term anti-herpetic activity, while the zinc containing compound provides the shorter term anti-herpetic activity.

Preferably, zinc oxide is present in compositions of the invention at levels of from 0.01%, more preferably from 0.1%, most preferably from 0.5%, particularly preferred from 1.0% and preferably up to 20%, more preferably up to 10%, most preferably up to 7%, and particularly preferred is up to 5% by weight of the composition.

### Flavoring agent

Compositions comprising zinc may cause distorted taste in certain subjects, as for instance described in EP987021. We have found that flavoring agents can be stably - physically and/or chemically- incorporated in compositions of the invention that comprise zinc containing compounds in a hydrophobic phase, leading to low or no side-effects while providing excellent lip feel.

Preferably, flavoring agents are selected from natural and synthetic flavors and may include fruit, plant and/or herbal extracts (preferably hydrophobic). Examples include almond, aloe vera, anise, chamomile, cinnamon, citronella, citrus unshui, ginger, grapefruit, grape seed, green tea, licorice, menthol, mint lemon, lemongrass, lime, orange, peppermint, rosemary, sage, spearmint, strawberry, tangerine, thyme, vanilla, and/or willow bark. For optimal incorporation, flavoring agents are preferably anhydrous and preferably have a hydrophobic base.

Preferably flavoring agents are included in the compositions of the invention at a level of from 0.05%, more preferably from 0.1%, and preferably up to 2%, more preferably up to 1% by weight of the composition.

### Coloring agents

Compositions of the present invention preferably comprise a coloring agent. For instance compared to general dermatological products, lip compositions have strict color requirements. The coloring agents also preferably physically and/or chemically stably incorporated. Preferably, compositions of the invention comprise one or more coloring agents. Preferably, the coloring agent is selected from dyes (preferably hydrophobic), pigments, and mixtures thereof. It is within the skill in the art to choose coloring agents; examples are disclosed in the C.T.F.A., International Cosmetic Ingredient Dictionary and Handbook, 10the Edition, 2004.

Preferably, lip compositions of the invention comprise one or more coloring agents. Lipstick compositions preferably comprise at least one dye in combination with at least one pigment; lip balm compositions preferably comprise at least one dye; and lip gloss compositions preferably comprise at least one dye with at least one pigment (preferably pearlescent pigment).

Examples of fat-soluble dyes are Carmine C1022-25, Sudan red, DC Red 17, DC Green 6, beta-carotene, soybean oil, Sudan brown, DC Yellow 11, DC Violet 2, DC Orange 5, quinoline yellow, and annalto. Preferably, the dye is represent of from 0.01 to 5%. Preferably, pigments are selected from inorganic, organic or mixtures thereof. Examples of inorganic pigments include titanium or zinc dioxide, optionally treated at the surface, zirconium or cerium oxides, iron or chromium oxides, manganese violet, ultramarine blue, chromium hydrate and ferric blue. Examples of organic pigments include carbon black, pigments of D & C type and lakes based on cochineal carmine or on barium, strontium, calcium or aluminum. Pearlescent pigments (sometimes called nacres or iridescent particles; for the purpose of this invention included under pigments) can be chosen from white pearlescent pigments, such as mica covered with titanium or with bismuth oxychloride, colored pearlescent pigments, such as titanium oxide-coated mica with iron oxides, titanium oxide-coated mica with in particular ferric blue or chromium oxide, or titanium oxide-coated mica with an organic pigment of the abovementioned type, and pearlescent pigments based on bismuth oxychloride. Preferably, pigments can represent from 0.005%, more preferably from 0.5% and preferably up to 30%, more preferably up to 5%, most preferably up to 3% by weight of the composition. Typical amounts of pigment are from 0.2% to 1.5% by weight.

Preferably, coloring agents are included in the compositions of the invention at a level of from 0.1 more preferably from 1%, most preferably from 2%, and preferably up to 30% more preferably up to 10% most preferably up to 5% by weight of the composition.

### Other ingredients

Compositions of the invention may comprise solid particles. Examples of solid particles are zinc oxide, titanium oxide, and pigments. Preferably, solid particles are present at a level of from 0.01 %, more preferably of from 0.1%, most preferably from 0.5%, particularly preferred from 1.0% and preferably up to 20%, more preferably up to 10%, most preferably up to 7%, and particularly preferred is up to 5% by weight of the composition.

Preferably, the solid particles are stably suspended in the composition with a structurant rendering the composition physically stable. Structurants are preferably selected from waxes (creating a solid phase in which the particles can be stably suspended), lamellar structures (for instance created by wax-oil combinations, in which the particles can be stably suspended; for example Polyglyceryl-3-Beeswax, PEG-8-Beeswax, stearyl Beeswax; these lamellar structurants are preferred in that they may improve bioavailability of the components, especially the active ingredients, of the composition), polymers (such as Kraton polymer, for instance Kraton G1701 E and Kraton G1650 E) and mixtures thereof. Preferably, polymers are present at a level of from 0.1%, more preferably of from 1%, and preferably up to 10%, more preferably up to 5% by weight of the composition.

Compositions may comprise vitamin compounds, precursors, and derivatives thereof. These vitamin compounds may be in either natural or synthetic form, for instance vitamin A, B, C, D, E and/or K at levels of from 0.000001, preferably from 0.1% up to 2% preferably 1% by weight of the composition.

Optionally, water present and, if present, the composition is preferably a water in oil composition. Preferably the compositions according to the invention comprise from 0% to 15% by weight of water, more preferably from 0 to 10% of water, most preferably from 0% to 5%; particularly preferred are compositions comprising less than 0.5% by weight of water. Most particularly preferred are compositions comprising substantially no water (i.e. 0% by weight of the composition), thus non-aqueous or anhydrous compositions. Without wishing to be bound by any theory, it is believed that low or no water may lead to lower wash-off effects, high bioavailability and great efficacy.

### Compositions

Compositions according to the invention preferably are selected from lipstick, lip balm, and lip gloss.

According to one aspect of the present invention, the lip composition is a lipstick. Preferably, the lipstick is solid at room temperature (20°C). Preferably, the lipstick comprises solid particles at a level of from 0.1%, more preferably of from 1% and preferably up to 50%, more preferably up to 30% by weight of the composition. The lipstick comprises preferably wax at a level of from 0.1%, more preferably from 3%, most preferably from 7% and preferably up to 30%, more preferably up to 25%, most preferably up to 20% by weight of the composition. Lipsticks according to the invention preferably comprise pigments at a level of from 0.1%, more preferably from 2% and preferably up to 50% and more preferably up to 30% by weight of the composition.

According to another aspect of the present invention, the lip composition is represented by a lip balm. Preferably, the lip balm is solid at room temperature (20°C). Lip balm generally comprise medication, for instance to prevent or treat chapped or sunburned lips. Preferably, the lip balm comprises solid particles at a level of from 0.1%, more preferably of from 1% and preferably up to 50%, more preferably up to 30% by weight of the composition. The lip balm comprises preferably wax at a level of from 0.1%, more preferably from 2%, most preferably from 4%, and preferably up to 15%, more preferably up to 12%, most preferably up to 7% by weight of the composition.

According to another aspect of the present invention, the lip composition is a lip gloss. Preferably, lip gloss is semi-solid at room temperature (20°C). Preferably, the lip gloss comprises solid particles at a level of from 0.1%, more preferably of from 1% and preferably up to 50%, more preferably up to 30% by weight of the composition. Preferably, the solid particles comprise pearlescent pigment, more preferably at levels of from 0.1 %, more preferably from 2% and preferably up to 50% and more preferably up to 30% by weight of the composition. The lip gloss comprises preferably wax at a level of from 0.1%, more preferably from 2%, most preferably from 4%, and preferably up to 15%, more preferably up to 12%, most preferably up to 7% by weight of the composition. Lip gloss is (in comparison with lip balm) less viscous, and generally comprises pearlescent pigment (glitters) and/or coloring agent. Lip gloss tends not to be medicated but, instead, is used for an esthetically pleasing look to the lips.

Lip creams are generally distinct from lipsticks, lip balm and lip gloss by having an aqueous continuous phase, while the present invention is preferably (but not exclusively) directed at a hydrophobic continuous phase, potentially allowing for better and/or quicker bioavailability of the compounds in the composition.

Preferably, the composition comprising the zinc containing compound dissolved in the hydrophobic phase is solid at a temperature of from -5 to 50°C, more preferably from 0-40°C, most preferably up to 37°C.

Preferably, zinc oxide is present in compositions of the invention at levels of from 0.01 %, more preferably from 0.1%, most preferably from 0.5%, particularly preferred 1.0% and preferably up to 20%, more preferably up to 10%, most preferably up to 7%, and particularly preferred is up to 5% by weight of the composition.

Preferably, compositions according to the invention are solid. For the purpose of the invention, compositions are defined to be solid if they have a viscosity of 50k cPs Brookfield (depending on the spindle temperature and rotational speed used, and also configuration dependent). Semi-solid formulations, such as lip gloss, are (accordingly) also defined as solid according to the invention.

### Composition preparation

Preferably, compositions of the present invention are prepared by dissolving the zinc containing compound in the hydrophobic phase at elevated temperature.

The invention is directed to a method of preparing a composition comprising a hydrophobic phase and a zinc containing compound, wherein the zinc containing compound is dissolved in the hydrophobic phase at an elevated temperature and wherein the composition is cooled down. Preferably, the composition comprises from 0-15% by weight of water.

In order to obtain the preferred dissolution characteristics (for instance in terms of level and/or speed for preparation of the formulations according to the invention), the zinc containing compound is preferably dissolved in -at least part of (preferably at least 50% by weight) - the hydrophobic phase of the invention at an elevated temperature, more preferably at a temperature of from 40°C and more preferably up to 130°C, most preferably from 65°C, in particularly preferred from 80°C, and even more particularly preferred from 85°C and most preferably up to 100°C and in particularly preferred up to 90°C.

Subsequently (to dissolution in the hydrophobic phase), the mix of the zinc containing compound and the hydrophobic phase is preferably cooled down. Preferably, the mix is homogenized during cooling down. Preferably, the mix is cooled down until solid and/or semi-solid, more preferably solid.

Preferably, compositions of the invention have a temperature of 50°C or lower, more preferably of 40°C or lower (and preferably -5 °C or higher, more preferably 0 °C or higher) in order to be applied on the lip. Preferably, the compositions are solid at this temperature, for instance room temperature (i.e. 20°C).

Preferably, flavoring agents are added to the mix at a temperature of from 30°C to 70°C, more preferably from 40°C to 65°C.

Preferably, the composition is filled in a container at a temperature of from 30°C and up to 65°C, more preferably from 40°, most preferably from 45°C and more preferably up to 60°C, most preferably up to 55°C.

### Herpetic infections on the lip

Herpes viruses, particularly herpes simplex and especially Herpes labialis viruses can cause cold sores on lips. The invention is directed at use of a zinc containing compound dissolved in a hydrophobic phase for prevention and/or treatment of perioral sore, particularly lip sores, more particularly viral infection (for instance a herpetic infections), most particular of herpetic cold sore infections on the lip, sometimes also called fever blisters.

As indicated above, we have surprisingly found that compositions of the present invention are physically and/or chemically stable, yet provide excellent prevention and efficacy against cold sores on the lip. Without wishing to be bound by any theory, it is believed that the low wash-off effect of the hydrophobic phase as well as the dissolved status of the zinc containing compound lead to the benefits of the invention in prevention and/or treatment of herpetic infections.

Lips are clearly distinct from general skin. For instance, lips do not contain sweat glands, hair, nor sebum, making them more sensitive than general skin. These differences have consequences for the requirements for treatment and for composition formulation. For instance, lips have higher composition wash-off effects, which requires frequent reapplication of the lip compositions. Further, lip compositions preferably meet strict requirements for taste, look, and feel for the user. Compositions of the present invention provides these benefits to the user of our lip compositions while providing prevention and/or treatment of herpetic infections.

Preferably, the invention is directed to use of zinc containing compounds dissolved in a hydrophobic phase in the prevention and/or treatment of virus infections.

### Dispenser device

Preferably, compositions according to the present invention are used as solid solutions in a dispenser device with a lid. The solid solution may be applied on the lip.

Accordingly, the present invention is directed to a dispenser device comprising a lid and a reservoir, wherein the reservoir comprises a composition comprising a zinc containing compound dissolved in a hydrophobic phase.

The reservoir of the invention may be a container, a tube or a jar. Lipstick may be provided in a piston tube container. Lip balm may be provided in a jar or in a piston tube container. Lip gloss may be provided in a tube container or jar. The compositions may be applied by hand or with an applicator, for instance a piston-driven dispenser device. Preferably, the present invention is directed at a method of manufacturing a dispenser device with a composition comprising a zinc containing compound dissolved in a hydrophobic phase , wherein the device is filled with the composition at a temperature of from 30°C and up to 65°C. Preferably, the dispenser is filled at a temperature of from 30°C and up to 65°C, more preferably from 40°, most preferably from 45°C and more preferably up to 60°C, most preferably up to 55°C.

### Examples

The following lip balm formulation was prepared (representing the preferred embodiment of the present invention):

| Ingredients | % by weight |
|---|---|
| jojoba oil | 74.17 |
| polymer* | 3.93 |
| zincoxide | 2.2 |
| PEG-8-beeswax | 16 |
| zinc ricinoleate | 2 |
| moisturizing and flavoring mix** | 1.7 |

| | |
|---|---|
| * 95:1 mix of Kraton G1701 E and Kraton G1650 E ** vitamine E (at 0.65%), shea butter (at 0.35%), dye (at 0.1%) and spearmint flavor (at 0.05%) | |

The polymer mix was mixed and dissolved in 62% of all jojoba oil at 90°C and microfine zincoxide was mixed in at 85 °C. The remaining jojoba oil, the PEG-8-Beeswax and zinc ricinoleate were separately mixed at 85-90°C (resulting in a fully clear, low viscous and transparent phase) and slowly added to the first jojoba mix at 90°C, leading to a homogeneous, low viscous opaque liquid that was gradually cooled (if necessary) under rehomogenizing. Moisturizing ingredients were added around 70°C and flavoring ingredients around 55°C. At 45-50°C, the formulation became slightly hazy and was filled in a lip balm jar.

The resulting lip balm composition was physically and chemically stable, allowed for easy and flexible processing, and requires minimal grinding. Applying the lip balm was easy with minimal wash-off effect and without astringent effects or gritty feel, creating excellent kiss appeal. The balm provided for good prophylactic and therapeutic efficacy against Herpes viruses.

## Claims

1. Composition comprising a zinc containing compound dissolved in a hydrophobic phase.

2. Composition according to claim 1, wherein the zinc containing compound is selected from zinc ricinoleate, zinc acetylacetonate, zinc gluconate, zinc undecylenate, zinc stearate, zinc palmitate, zinc succinate, zinc lactate, zinc pyrrolidon, zinc bis-(8-hydroxycaprylate), zinc glutamate, zinc malate and zinc tartrate.

3. Composition according to claims 1-2 wherein the composition comprises from 0.1% to 30% by weight of a wax.

4. Composition according to claims 1-3 wherein the composition comprises a flavoring agent.

5. Composition according to claim 1-4, wherein the composition comprises a coloring agent.

6. Composition according to claims 1-5, wherein the composition comprises a hydrophobic continuous phase and from 0% to 15% by weight of water.

7. Composition according to claims 1-6, wherein the composition comprises from 0.01% to 20% by weight of solid particles.

8. Composition according to claim 1-7, wherein the composition comprises a from 0.01% to 20% by weight of zinc oxide.

9. Composition according to claims 1-8, wherein the composition is a lipstick, lip balm or lip gloss and wherein the composition is solid at a temperature of 20°C

10. Method of preparing a composition comprising a hydrophobic phase and a zinc containing compound, wherein the zinc containing compound is dissolved in the hydrophobic phase at an elevated temperature, wherein the composition is cooled down and wherein the composition comprises less than 15% by weight of water.

11. Use of zinc containing compounds dissolved in a hydrophobic phase in the treatment or prevention of lip sores.

12. Use according to claim 11, wherein the lip sore is a viral infection.

13. Dispenser device comprising a container reservoir and a lid, wherein the reservoir comprises a composition comprising a zinc containing compound dissolved in a hydrophobic phase.

14. Method of manufacturing a dispenser device with a composition comprising a zinc containing compound dissolved in a hydrophobic phase , wherein the device is filled with the composition at a temperature of from 30°C and up to 65°C.

15. Composition comprising zinc containing compound and zinc oxide particles.
